# EUROPEAN PATENT SPECIFICATION

(11) **EP 1 616 945 B1**
(45) Date of publication and mention of the grant of the patent: **21.11.2007**
(21) Application number: 05021429.5
(22) Date of filing: 26.12.1995
(51) Int. Cl.: C12N 9/86, C12N 15/00, C12P 41/00

(54) **Process for producing D-N-carbamoyl-alpha-amino acids**
Verfahren zur Herstellung einer D-N-Carbamoyl-alpha-Aminosäure
Procédé de fabrication de l'acide D-N-carbamyle-alpha-amine

(30) Priority: 28.12.1994 JP 32686594
(43) Date of publication of application: 18.01.2006
(62) Divisional of application: 95941892.2
(73) Proprietor: KANEGAFUCHI KAGAKU KOGYO KABUSHIKI KAISHA, Kita-ku Osaka-shi Osaka-fu 530 (JP)
(72) Inventor: Nanba, Hirokazu, Takasago-shi, Hyogo 676-0023 (JP); Yajima, Kazuyoshi, Akashi-shi, Hyogo-ken (JP); Takano, Masayuki, Kakogawa-shi, Hyogo-ken (JP); Yamada, Yukio, Kakogawa-shi, Hyogo-ken (JP); Ikenaka, Yasuhiro, Kobe-shi, Hyogo 651-2242 (JP); Takahashi, Satomi, Kobe-shi, Hyogo-ken (JP)
(74) Representative: HOFFMANN EITLE

(56) References cited:
- EP-A- 0 543 021
- EP-A- 0 602 247
- WO-A-94/00577

## Description

### Field of the Invention

The present invention relates to a process for producing D-N-carbamoyl-α-amino acids. More specifically, it relates to a process for producing D-N-carbamoyl-α-amino acids utilizing a novel transformant having a gene for an enzyme which converts 5-substituted hydantoins into corresponding D-N-carbamoyl-α-amino acids by asymmetric cleavage hydrolysis (hereinafter referred to as hydantoinase).

### Backgrounds of the Invention

Optically-active D-N-carbamoyl-α-amino acids can be converted into corresponding D-α-amino acids and optically-active D-α-amino acids are important compounds as intermediate compounds for drugs. In particular, examples of industrially useful compounds include D-phenylglycine and D-(p-hydroxyphenyl)glycine as intermediate compounds for the production of semisynthetic penicillins and semisynthetic cephalosporins.

For the production of D-N-carbamoyl-α-amino acids, processes utilizing microbial enzymatic reactions have been known to convert 5-substituted hydantoins into corresponding D-N-carbamoyl-α-amino acids by asymmetric cleavage hydrolysis and such processes are described in JP-A 53-91189, JP-A 53-44690, JP-A 53-69884, JP-A 53-133688 and the like.

In addition, a gene relating to hydantoinase which is an enzyme for conventing a 5-substituted hydantoin into the corresponding D-N-carbamoyl-α- amino acid is obtained
from thermophilic microorganisms and it is inserted into mesophilic microorganisms to produce transformant microorganisms having hydantoinase activity. Furthermore, in WO94/00577, a hydantoinase gene fragment is isolated from a microorganism belonging to the genus Agrobacterium and introduced into Escherichia coli or a microorganism of the genus Agrobacterium to express the activity.

### Objects of the Invention

In the above-described processes utilizing microbial enzymatic reactions, there are such drawbacks that yields of the enzyme by microorganisms known to be its source is insufficient and, further, culture mediums are expensive.

In addition, with regard to the transformant microorganisms disclosed in JP-A 62-87089, the improvement of the enzymatic activity is only several times in comparison with sporulating thermophilic microorganisms having hydantoinase activity and yields of the enzyme is yet insufficient.

Furthermore, there is no example in the prior art that a 5-substituted hydantoin is subjected to asymmetrical cleavage hydrolysis by using the above-described transformant microorganism to produce and accumulate the corresponding D-N-carbamoyl-α-amino acid.

The present invention is to solve these problems and is directed to the creation of a microorganism having very high enzyme productivity and to the production of D-N-carbamoyl-α-amino acids efficiently by using the enzyme source thus obtained.

### Summary of the Invention

JP-A 62-87089 and WO94/00577 disclose the method for obtaining hydantoinase producer microorganisms by using recombinant DNA techniques. However, the source microorganisms for supplying hydantoinase genes are completely different from microorganisms used for obtaining hydantoinase genes in the present invention. In addition, the nucleotide sequences and amino acid sequences of the hydantoinase genes are considerably different from those of the present invention.

The present invention is to provide a process for producing a D-N-carbamoyl-α-amino acid which comprises reacting a 5-substituted hydantoin in an aqueous medium with hydantoinase produced by a transformant obtained by transforming a host microorganism such as a microorganism belonging to the genus Escherichia, Pseudomonas, Flavobacterium, Bacillus, Serratia, Agrobacterium, Corynebacterium or Brevibacterium with a recombinant DNA of a DNA fragment containing a gene for hydantoinase derived from Bacillus sp. KNK245 (FERM BP-4863) (not part of the invention), Agrobacterium sp. KNK712 (FERM BP-1900) or Pseudomonas sp. KNK003A (FERM BP-3181) (not part of the invention) and a vector DNA.

A transformant having very high hydantoinase productivity such as that obtained in the present invention has not been known heretofore in the prior art and the extremely efficient and economical production of D-N-carbamoyl-α-amino acids become possible by utilizing the reaction of the enzyme of the transformant.

Hereinafter, the present invention will be described in detail with reference to the accompanying drawings.

### Brief Explanation of the Drawings

Fig. 1 is a restriction enzyme map of plasmid pAH1043.
Fig. 2 is a restriction enzyme map of plasmid pTH102 (not part of the invention)
Fig. 3 is a restriction enzyme map of plasmid pTH103 (not part of the invention)
Fig. 4 is a restriction enzyme map of plasmid PTH104 (not part of the invention)
Fig. 5 is a restriction enzyme map of plasmid pPHD301 (not part of the invention)
Fig. 6 is a restriction enzyme map of plasmid PTHB301 (not part of the invention).

### Detailed Explanation of the Invention

A DNA fragment containing the gene to be used in the present invention can be obtained from Bacillus sp. KNK245 (not part of the invention), Agrobacterium sp. KNK712 or Pseudomonas sp. KNK003A. (not part of the invention). Agrobacterium sp. KNK712 and Pseudomonas sp. KNK003A (not part of the invention) have been deposited at National Institute of Bioscience and Human-Technology, Agency of Industrial Science & Technology (NIBH), Ministry of International Trade & Industry by the present applicant under accession numbers of FERM BP-1900 and FERM BP-3181, respectively and are known strains which described in detail in WO92/10579. Bacillus sp. KNK245 (not part of the invention) is a strain newly found by the present inventors and has been deposited at NIBH as of November 2, 1994 under the accession number of FERM BP-4863.

Microbiological characteristics of Bacillus sp. KNK245 are as follows.

**Bacillus sp. KNK245**

| Form | | rods, filamentous |
|---|---|---|
| Size | | 0.5-0.8 x 3-10 (µm) |
| Gram stain | | + |
| Spores | | + |
| Motility | | - |
| Growth temperature | | 37-60°C |
| Growth pH | | 5.0-8.9 |
| Growth in 5% NaCl | | - |
| Growth in 7% NaCl | | - |
| Growth in 0.02% azide | | - |
| Growth in 0.001% lysozyme | | - |
| Nitrate reduction | | - |
| Starch hydrolysis | | + |
| Casein decomposition | | + |
| Tyrosine decomposition | | - |
| Catalase | | - |
| Oxidase | | + |
| Indole production | | - |

| Production of acids from carbohydrates | | |
|---|---|---|
| | Mannose | (+) |
| | Xylose | - |
| | Arabinose | + |
| | Rhamnose | - |
| | Glucose | + |
| | Fructose | + |
| | Raffinose | - |
| | Sucrose | + |
| | Maltose | + |
| | Lactose | - |

For obtaining a hydantoinase gene from these strains, normally, the following procedure is carried out.

Firstly, genomic DNA is extracted from a microbial cell and then the DNA is partially hydrolyzed with a suitable restriction enzyme, for example, Sau3AI or the like. The resulting fragments are ligated to vector DNA to obtain recombinant DNA molecules having various genomic DNA fragments as a gene library (see JP-A 58-126789 and U.S. Patent No. 4,237,224).

Then, a recombinant containing the desired gene is selected from this gene library. For example, the selection can be carried out by detection of an expressed protein using its enzymatic activity as an indication, or the selection can be carried out by analyzing the N-terminal sequence of a protein, synthesizing a corresponding DNA probe and detecting the presence of the desired gene by colony hybridization or the like. In addition, the desired gene can be obtained by colony hybridization or polymerase chain reaction (PCR) method using DNA primers which are synthesized suitable portions of a known hydantoinase base sequence. Once the desired gene is obtained, its nucleotide sequence is analyzed. In addition, a hydantoinase producer microorganism and a recombinant containing this enzyme gene are cultivated. The resultant enzyme is purified and the molecular weight of the resultant protein is determined. At the same time, the amino acid sequence about its N-terminal region is determined with a gas phase protein sequencer or the like.

Then, by comparison of these DNA nucleotide sequences and N-terminal amino acid sequences, the initiation site for translation of the nucleotide sequence portion encoding a hydantoinase protein into the protein is determined and, by considering the relation to the molecular weight of the protein, it is confirmed that the enzyme protein is encoded in the gene portion extending from this site to the termination codon, thereby verifying the desired gene (Molecular Cloning, A Laboratory Manual, 2nd Ed., Cold Spring Harbor Laboratory Press, Chapters 4 and 13). Thus, DNA fragments of SEQ ID Nos. 1 and 3 were obtained from Agrobacterium sp. KNK712 and Pseudomonas sp. KNK003A (not part of the invention). As is well known, the gene encoding the enzyme thus obtained and/or DNA fragment containing it are equivalent to DNA fragments having another nucleotide sequence which encodes an amino acid sequence corresponding to the above gene and/or DNA fragment because one amino acid usually corresponds to a plurality of base codons.

As the vector to be used in the present invention, plasmids, phages or derivatives thereof can be used, which are derived from microorganisms and can be autonomously grown in cells of bacteria belonging to the genus Escherichia, Pseudomonas, Flavobacterium, Bacillus, Serratia, Corynebacterium or Brevibacterium.

For example, host-vector systems described in "Guidelines on Recombinant DNA Experiments, -Commentation, Q and A-" (the Life Science Section of the Research Development Office in the Science and Technology Agency, Ed., revised September 16, 1987), pages 25 to 27 and 36 to 38 can be used. In addition, recombinant DNA having a translation initiation site linked to a suitable position of a vector which has been modified to have a strong structural promoter can be used for the purpose of increasing the amount of enzyme to be produced. Both ends of these fragments are cleaved with a restriction enzyme to produce a recombinant with an appropriate vector. A hydantoinase producer recombinant microorganism can be produced by directly transforming a microorganism belonging to the genus Escherichia, Pseudomonas, Flavobacterium, Bacillus, Serratia, Agrobacterium, Corynebacterium or Brevibacterium.

A hydantoinase producer recombinant microorganism can also be obtained without direct transformation of a microorganism of the above-described genus; that is, the desired gene is once cloned in a host vector system using other microorganisms such as Escherichia coli, after which recombinant DNA with an appropriate vector is produced.

The description of the following documents can be widely applied to the production of recombinants: the specification of U.S. Patent No. 4,237,244 to S. N. Cohen et al.; "Idenshi-sousa Jikken-hou (Experimental Method of Gene Manipulation)" [Ed., Yasuyuki TAKAGI, Kohdan-sha Scientific (1980)]; Method in Enzymology, 68, Recombinant DNA [Ed., Ray Mv, Academic Press (1979)], the specification of JP-A 58-126789 and the like.

The desired gene is cloned and unnecessary DNA thereof is removed. Recombinant DNA having its translation initiation site linked to a suitable position of a vector which has been modified to have a strong structural promoter can be used for transformation of the above host cell to increase the amount of the enzyme to be produced.

The transformant is cultivated in a conventional nutrient culture medium to express the introduced recombinant DNA. When a character derived from gene DNA or vector DNA is conferred to the recombinant DNA, an appropriate component can be added to the culture medium according to a particular character.

To take the transformant thus obtained as a source of enzyme supply, it can be cultivated by using the conventional culture medium. If necessary, it is also possible to carry out a treatment for enzyme induction such as an addition of hydantoin compounds, uracil, isopropyl-1-thio-β-D-galactosides (IPTG) or the like and a temperature increase. Usually, the culture medium used for cultivating the transformant can be a conventional culture medium containing carbon sources, nitrogen source and inorganic ions. In many cases, desired results can be obtained by further adding organic trace nutrients such as vitamins, amino acids and the like. As the carbon sources, carbohydrates such as glucose and sucrose, organic acids such as acetic acid, alcohols and the like can be conveniently used. As the nitrogen sources, ammonia gas, ammonia water, ammonium salts and the like can be used. As the inorganic ions, phosphate ion, magnesium ion, potassium ion, iron ion, manganese ion, cobalt ion, nickel ion, copper ion, aluminum ion, molybdenum ion and the like can be used.

If cultivation is carried out under aerobic conditions for 1 to 10 days, while adjusting the pH and temperature to an appropriate range of 4 to 8 and 25 to 60°C, respectively, desired results can be obtained.

The enzymatic activity of an enzyme produced by the transformant can be exhibited in the form of, for example, a culture solution of the transformant, its microbial cells, the enzyme extracted from the microbial cells, immobilized microbial cells and the like.

As the microbial cells, any of the culture solution as it is after completion of cultivation, microbial cells separated from the culture solution, washed microbial cells and the like can be used. As the treated microbial cells, lyophilized microbial cells, acetone-dried microbial cells, microbial cells brought into contact with toluene or a detergent, lysozyme treated microbial cells, ultrasonicated microbial cells, mechanically ground microbial cells and the like can be used. In addition, enzyme extracts having hydantoinase activity obtained from these treated microbial cells, these immobilized microbial cells, insolubilized treated microbial cells, enzyme proteins fixed on a support for immobilization (e.g., anion exchange resin) and the like can be used. For an immobilization method, for example, the specification of JP-A 63-185382 and the like can be referred to.

As the support used for immobilization, suited are phenol-formaldehyde anion exchange resins such as Duolite A568 or DS17186 (Rohm & Haas Co.: registered trade mark); and various anion exchange resins containing various amines, ammonium salts, or functional groups of the diethanolamine type, for example, polystylene resins such as Amberlite IRA935, IRA945, IRA901 (Rohm & Haas Co.: registered trade mark), Lewatit OC1037 (Bayer A.G.: registered trade mark) and Diaion EX-05 (Mitsubishi Chemical Industries, Ltd.: registered trade mark). Other supports such as DEAE-cellulose can also be used.

Further, in order to obtain stronger and more stable adsorption of enzymes, usually, cross-linking agents are used, the preferred examples thereof being glutaraldehyde. As the enzyme to be used, not only purified enzymes, but also those with different degrees of purification such as partially purified enzymes, suspensions of disintegrated microbial cells and cell-free extracts can be used. The preparation of immobilized enzymes can be carried out according to a conventional method, for example, by adsorbing enzymes on a support, followed by cross-linking treatment.

The 5-substituted hydantoin to be used as a substrate of the enzymatic reaction of the present invention is not specifically limited and can be any compound normally used in this kind of reaction. Examples thereof include that represented by the general formula (1):

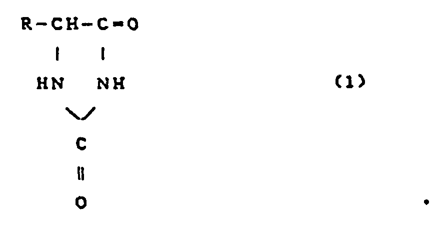

The substituent R in the compound of the formula (1) can be selected in a wide range. In order to provide industrially useful products such as intermediate compounds of drugs, preferably, R is phenyl, phenyl substituted with hydroxy, alkyl, substituted alkyl, aralkyl or thienyl. In the case of phenyl substituted with hydroxy, the number of hydroxy may be one or more and they can be located at any of o-, m- and p-positions, with the typical example thereof being p-hydroxyphenyl. The alkyl group is such a group of 1 to 4 carbon atoms that the corresponding D-N-carbamoyl-α-amino acid is D-N-carbamoyl-alanine, D-N-carbamoyl-valine, D-N-carbamoyl-leucine, D-N-carbamoyl-isoleucine or the like. The substituted alkyl group is such an alkyl group of 1 to 4 carbon atoms substituted with hydroxy, alkylthio, carboxyl, amino, phenyl, phenyl substituted with hydroxy, amido or the like that the corresponding D-N-carbamoyl-amino acid is D-N-carbamoyl-serine, D-N-carbamoyl-threonine, D-N-carbamoyl-methionine, D-N-carbamoyl-cysteine, D-N-carbamoyl-asparagine, D-N-carbamoyl-glutamine, D-N-carbamoyl-tyrosine, D-N-carbamoyl-tryptophane, D-N-carbamoyl-aspartic acid, D-N-carbamoyl-glutamic acid, D-N-carbamoyl-histidine, D-N-carbamoyl-lysine, D-N-carbamoyl-arginine, D-N-carbamoyl-citrulline or the like. The aralkyl group is such a group having 7 to 8 carbon atoms, for example, benzyl or phenethyl, that the corresponding D-N-carbamoyl-α-amino acid is D-N-carbamoyl-phenylalanine or the like.

As the aqueous medium, those containing water, buffers or organic solvents such as ethanol can be used. Further, when necessary, nutrients required for the growth of microorganisms, antioxidants, detergents, coenzymes, hydroxylamines, metals and the like can also be added to the aqueous medium.

In the case where, while cultivating the microbial cells of the above microorganisms in a water-soluble medium, the microbial cells are brought into contact with a particular 5-substituted hydantoin, an aqueous medium containing not only the 5-substituted hydantoin but also nutrients required for the growth of microorganisms such as carbon sources, nitrogen sources and inorganic ions is used. If organic trace nutrients such as vitamins and amino acids are further added thereto, satisfactory results can be obtained in many cases. As the carbon sources, carbohydrates such as glucose, sucrose; organic acids such as acetic acid; alcohols and the like are conveniently used. As the nitrogen sources, ammonia gas, ammonia water, ammonium salts and the like can be used. As the inorganic ions, phosphate ion, magnesium ion, potassium ion, iron ion, manganese ion, cobalt ion, nickel ion, copper ion, aluminum ion, molybdenum ion and the like can be used.

The cultivation is carried out under aerobic conditions, while adjusting the pH and temperature to an appropriate range of 4 to 8 and 25 to 60°C, respectively. If the cultivation is carried out for 1 to 10 days, 5-substituted hydantoins are converted into only D-N-carbamoyl-α-amino acids with high efficiency.

On the other hand, in the case where the culture solution of the above-microorganisms is allowed as it is to react with cultivated microbial cells, treated microbial cells, enzyme extracts, immobilized microbial cells, insolubilized microbial cells or fixed enzyme proteins in an aqueous medium containing a 5-substituted hydantoin dissolved or suspended, the reaction mixture can be allowed to stand for some time or stirred, while maintaining at an appropriate temperature of 10 to 80°C and pH of 4 to 9.5. Thus, if 5 to 100 hours have run their course, D-specific hydrolysis reaction of the substrate by hydantoinase and chemical racemization of the substrate proceed and any of D-, DL- or L-5-substituted hydantoins are converted into the corresponding D-N-carbamoyl-α-amino acids to accumulate a large amount of the amino acids in the aqueous medium. In addition, 5-substituted hydantoins can be added in separate portions with the progress of reaction.

The produced D-N-carbamoyl-α-amino acid can be isolated and purified by a conventional separation method.

D-α-amino acids can be easily obtained by using the isolated and purified D-N-carbamoyl-α-amino acids obtained by the above-described method or the reaction mixtures obtained by the hydantoinase reaction as they are by the action and converting the D-N-carbamoyl-α-amino acids into the corresponding D-α-amino acids chemically or enzymatically with enzymes having decarbamylase activity.

The following Examples further illustrate the present invention in detail.

### Reference Example 1

### Isolation of microorganism having hydantoinase activity from soil

After suspending 0.5 g of a sample of soil in 2 ml of a physiological saline, the suspension was allowed to stand and one drop of the supernatant was smeared on an isolation agar plate medium (1.0% of meat extract, 1.0% of peptone, 1.0% of yeast extract, 0.3% of sodium chloride, 2.0% of agar; pH 7.5). This was cultivated at 50°C for 2 days and the colony obtained was transferred to the same isolation plate medium to which 0.1% of uracil and 20 ppm of manganese chloride and then further cultivated at 50°C for 1 day. The microbial cells thus obtained were suspended in 100 µl of a reaction substrate solution [DL-(p-hydroxyphenyl)hydantoin 30 mM, sodium sulfite 0.1%, carbonate buffer 50 mM] and they were reacted at 50°C for 2 hours. Then, the reaction mixture was spotted on a TLC plate and developed with a developing solvent of butanol : acetic acid : water (4 : 1 : 1), followed by developing color with a 10% solution of p-dimethylaminobenzaldehyde in conc. hydrochloric acid and detecting a yellow spot to select microbial strains producing N-carbamoyl-p-hydroxyphenylglycine. Thus, among 2740 colonies, Bacillus sp. KNK245 (FERM BP-4863) having high hydantoinase activity was isolated.

### Reference Example 2

### N-Terminal amino acid sequencing of hydantoinase derived from Bacillus sp. KNK245

A loopful of Bacillus sp. KNK245 (not part of the invention) was inoculated into 100 ml of a seed culture medium (1.0% of meat extract, 1.0% of peptone, 0.5% of yeast extract; pH 7.5) in a 500 ml Sakaguchi flask and cultivated at 55°C for 19 hours. Then, 2% thereof was inoculated into 500 ml of a main culture medium (1.0% of meat extract, 1.0% of peptone, 0.5% of uracil, 20 ppm of manganese chloride tetrahydrate; pH 7.5) in a 2 liter Sakaguchi flask and cultivated at 55°C for 19 hours. Microbial cells were collected from 9 liters of the culture broth thus obtained by centrifugation. They were suspended in 0.2 M Tris-HCl (pH 8.0) with 20 mM manganese sulfate, ultrasonicated and centrifuged to obtain a cell-free extract. Then, the extract was purified up to 32 times by means of ammonium sulfate precipitation fractionation, DEAE-Sepharose and phenyl-Sepharose. Further, hydantoinase crystals were obtained by using ammonium sulfate. The crystals were dissolved in water and analyzed by using 470 A Model Gas Phase Protein Sequencer (manufactured by Applied Biosystems). As a result, it was found that the hydantoinase had the amino acid sequence of: at its N-terminal.

### Example 1

To obtain plasmid containing gene for hydantoinase derived from Agrobacterium sp. KNK712 (FERM Bp-1900) It was found that a hydantoinase gene was located on the DNA fragment cloned in the plasmid pAHD101 described in WO92/10579 by a study according to the following method. Escherichia coli JM109 was transformed with pAHD101 according to a conventional method, inoculated into 50 ml of L-broth (10 g/liter of peptone, 5 g/liter of yeast extract, 5 g/liter of sodium chloride; pH 7.0) containing 100 mg/liter of ampicillin and cultivated at 37°C for 16 hours. Then, 50 ml of the culture broth was collected, followed by removing the supernatant, suspending the microbial cells in 50 ml of a substrate solution (0.5% of 5-(p-hydroxyphenyl)hydantoin, 0.05% of Triton X-100, 0.05 M phosphate buffer; pH 8.7) and reacting at 37°C for 3 hours in a stream of nitrogen. The reaction mixture was spotted on a TLC plate, developed and subjected to color development with a solution of p-dimethylaminobenzaldehyde in hydrochloride to detect a spot of D-N-carbamoyl-(p-hydroxyphenyl)glycine in agreement with an authentic sample. In view of the above, it was confirmed that the transformant of pAHD101 expresses a gene encoding a hydantoinase.

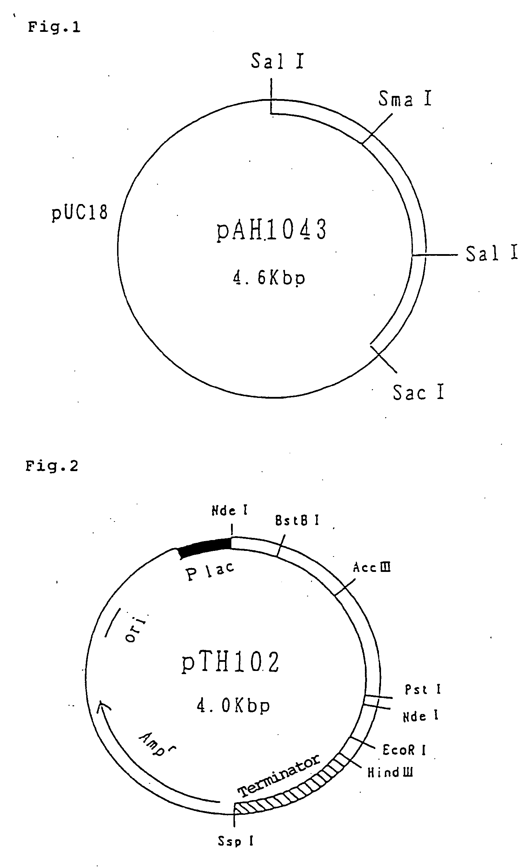

A 2.1 kbp fragment obtained by cleaving the plasmid pAHD101 with SacI and SalI to shorten the inserted fragment was ligated to pUC18 fragment cleaved by SacI and SalI to obtain the plasmid pAH1043 (see Fig. 1).

In addition, the nucleotide sequencing of the hydantoinase gene was carried out by using DNA sequence kit (manufactured by Applied Biosystems). The results are shown in SEQ ID No. 1

Escherichia coli HB101 was transformed with the plasmid pAH1043 to obtain a transformant, Escherichia coli HB101 pAH1043 (FERM BP-4865).

### Reference Example 3

### Preparation of recombinant DNA of genomic DNA of Bacillus sp. KNK245 and vector DNA

Bacillus sp. KNK245 (FERM BP-4863) was cultivated in 2 liters of a broth (10 g/liter of meat extract, 10 g/liter of peptone, 5 g/liter of yeast extract; pH 7.5) at 45°C for 24 hours and microbial cells were collected. Genomic DNA was extracted from the microbial cells obtained according to Marmur method. Ten units of BamHI was added to 1 µg of the genomic DNA and they were reacted at 37°C for 16 hours.

On the other hand, the plasmid pUC19 was completely cleaved with BamHI and ligated thereto the above-obtained genomic DNA fragment with T4DNA ligase to obtain a mixture of various recombinant plasmids.

### Reference Example 4

### To obtain hydantoinase gene derived from Bacillus sp. KNK245

Based on the nucleotide sequences of hydantoinase genes derived form Agrobacterium sp. KNK712 (FERM BP-1900) of Example 3 and Lu1220 strain disclosed in JP-A 62-87089, two kinds of primers 1 and 2 (see Table 1) having sequences of each complementary strand of the above nucleotide sequences oriented toward opposite directions from 1155 bp interrupting nucleotides were synthesized. A polymerase chain reaction (PCR) was carried out by using two primers which were synthesized by using genomic DNA derived from Bacillus sp. KNK245 prepared in Example 4 as a template to obtain about 1.2 kbp fragment. The nucleotide sequencing of this fragment was carried out by using DNA sequence kit (manufactured by Applied Biosystems). As a result, it had high complementation to a known hydantoinase gene and it was found that the PCR fragment obtained was a part of the hydantoinase gene. Then, two primers 3 and 4 (Table 1) having oppositely directed sequences of respective complementary strands of this fragment were synthesized.

Further, primer 5 (Table 1) having the sequence of the vector portion in the recombinant DNA of Example 4 was synthesized and, by using the above synthesized primers and the latter primer and using the recombinant DNA of Example 4 as a template, PCR was carried out to obtain two kinds of DNA fragments containing the front half and back half parts of the hydantoinase gene, respectively. These DNA fragments were subjected to nucleotide sequencing. Based on nucleotide sequences expected from the N-terminal amino acid sequence of the hydantoinase protein of Example 2, the translation codon was determined. Together with the results of the above-determined nucleotide sequences, the entire nucleotide sequence of the gene encoding hydantoinase was determined.

Then, based on the nucleotide sequence thus obtained, primer 6 (Table 1) having a sequence about the initiation codon of the hydantoinase gene and NdeI restriction site and primer 7 (Table 1) having a sequence about PstI restriction site in the hydantoinase gene were synthesized and PCR was carried out by using the genomic DNA of Example 4 as a template to obtain a 1.2 kb fragment 1. Then, primer 8 (Table 1) having an oppositely directed sequence at the above PstI restriction site and primer 9 (Table 1) having a sequence corresponding to the downstream of the termination codon and HindIII restriction site were synthesized and PCR was carried out by using the genomic DNA of Example 4 as template to obtain a 0.25 kb fragment 2. Fragment 1 was restricted with NdeI and PstI, fragment 2 was restricted with PstI and HindIII and pUCNT which was an improved vector plasmid of pUC19 described in WO94/03613 was restricted with NdeI and HindIII. The restricted fragments were ligated with T4DNA ligase to obtain plasmid pTH102 containing the hydantoinase gene (see Fig. 2).

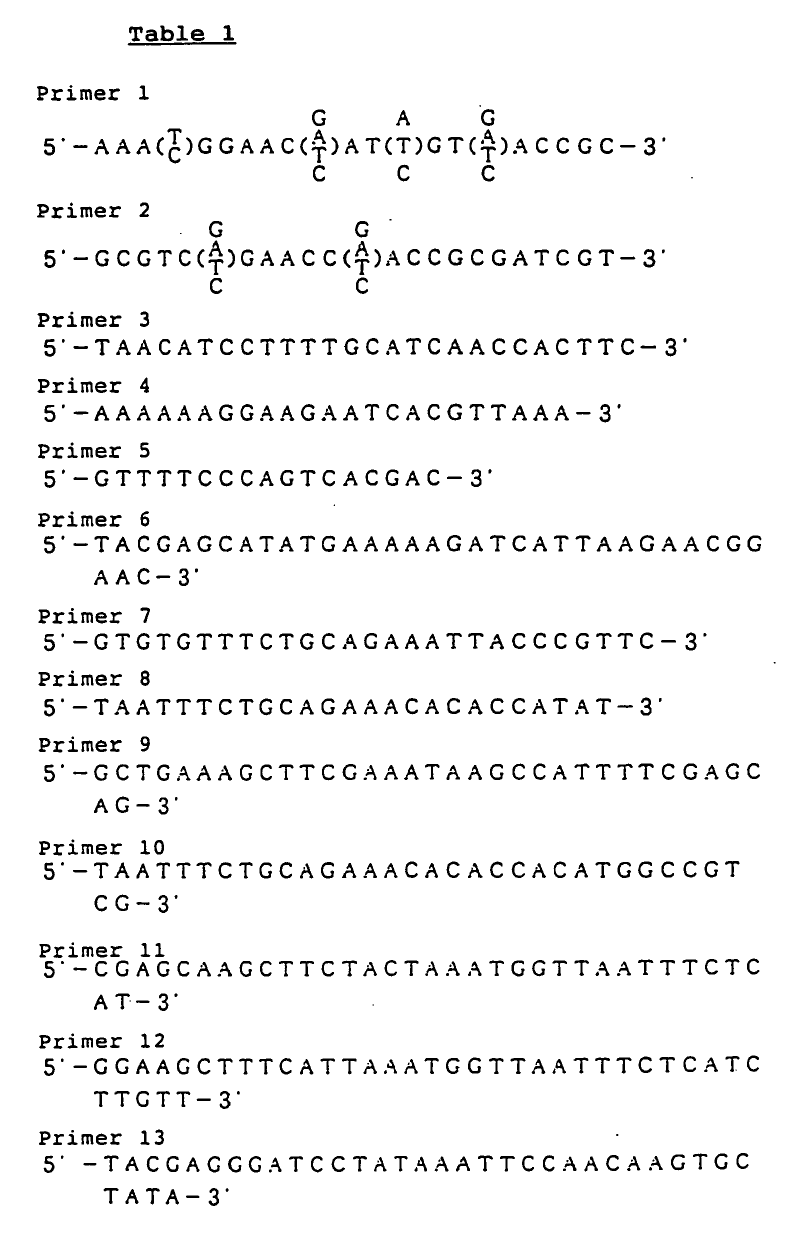

### Reference Example 5

### Preparation of recombinant DNA for expression of Bacillus sp. KNK245 hydantoinase gene

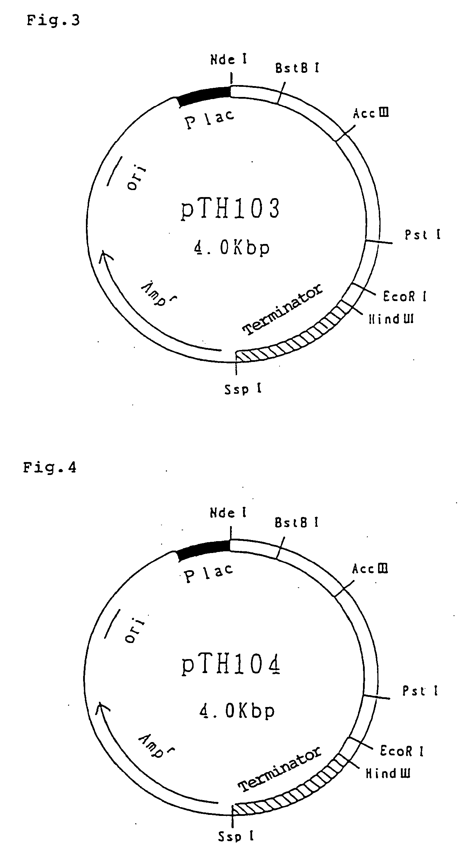

According to the same manner for obtaining fragment 2 in Reference Example 4, PCR was carried out to obtain a 0.25 kb fragment 3 except that, instead of the primer having PstI restriction site used for the production of fragment 2, synthesized primer 10 (Table 1) wherein one nucleotide at NdeI restriction site near PstI restriction site was substituted to block cleavage with NdeI was used and instead of the primer having the sequence about the termination codon, synthesized primer 11 (Table 1) wherein the number of nucleotides at the downstream of the termination codon were further reduced was used. Then, according to the same manner for obtaining pTH102 in Reference Example 4, the plasmid pTH103 was obtained from the fragments 1 and 3 and pUCNT (see Fig. 3).

Further, according to the same manner for obtaining pTH103, pTH104 was obtained except that, instead of the primer 11 having the downstream sequence about the termination codon, primer 12 (Table 1) was used.

Escherichia coli HB101 transformed with pTH104 was designated as Escherichia coli HB101 pTH104. This strain has been deposited at National Institute of Bioscience and Human-Technology as of November 2, 1994 under the accession number of FERM BP-4864.

### Reference Example 6

To obtain plasmid containing gene for hydantoinase derived from Pseudomonas sp. KNK003A (FERM BP-3181) According to the same manner as described in Example 1, it was found that hydantoinase gene was located on the plasmid pPHD301 described in WO92/10579.

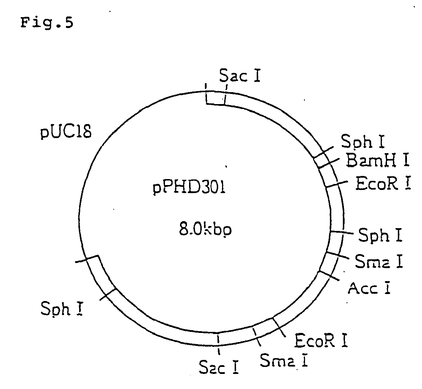

Escherichia coli HB101 was transformed by pPHD301. to obtain Escherichia coli HB101pPHD301 (FERM BP-4866). The restriction enzyme map of pPHD301 is shown in Fig. 5.

The nucleotide sequencing of the hydantoinase gene was carried out according to the same manner as described in Example 1. The results are shown in SEQ No. 3 (not part of the invention) SEQ No. 4 is also not part of the invention.

### Reference Example 7

### Conversion of 5-(p-hydroxyphenyl)hydantoin into D-N-carbamoyl-(p-hydroxyphenyl)glycine by transformant obtained

The transformant obtained in Reference Example 5, Escherichia coli HB101pTH104 was inoculated into 50 ml of 2YT broth (16 g/liter of polypeptone, 10 g/liter of yeast extract, 5 g/liter of sodium chloride; pH 7.0) containing 100 µg/ml of ampicillin and 400 ppm manganese chloride tetrahydrate and cultivated at 37°C for 24 hours. The microbial cells were collected from 50 ml of this culture broth and suspended in 0.05 M carbonate buffer (pH 8.7) to make up the volume to 50 ml. Triton X-100 was added so that the final concentration was 0.05%. To this was added 1.5 g of 5-(p-hydroxyphenyl)hydantoin and the mixture was allowed to react with stirring at 40°C for 3 hours in the stream of nitrogen with adjusting the pH at 8.7 by 6N sodium hydroxide. After completion of the reaction, the reaction mixture was centrifuged and the supernatant was subjected to a colorimetric determination of D-N-carbamoyl-(p-hydroxyphenyl)glycine therein by color development with 10% solution of p-diemthylaminobenzaldehyde in conc. hydrochloric acid. As a result, D-N-carbamoyl-(p-hydroxyphenyl)glycine was produced in the conversion yield of 97.5%.

The reaction mixture was adjusted to pH 5 with hydrochloric acid. After centrifugation, the supernatant was concentrated, adjusted to pH 2 with conc. hydrochloric acid and stored at 4°C. The precipitated crystals were filtered off and recrystallized from water-ethanol to obtain 970 mg of D-N-carbamoyl-(p-hydroxyphenyl)glycine, m.p. 176-177°C, [α]_{D}²⁰ = -177.0° (c=0.5, 5% ethanol).

### Reference Example 8

### Preparation of immobilized hydantoinase

Microbial cells were collected from 1 liter of a culture broth of Escherichia coli HB101pTH104 obtained according to the same manner as described in Reference Example 6, suspended in aqueous 1 mM manganese sulfate to make up the volume to 60 ml, adjusted to pH 8.5 and disrupted by ultrasonication. To the enzyme solution thus obtained was added 20 g of an anion exchange resin, Duolite A-568 equilibrated to pH 8.5 and the mixture was stirred at 15°C for 20 hours to adsorb the enzyme. To this mixture was added glutaraldehyde so that the final concentration thereof was 0.1% and the mixture was stirred for 1 hour to proceed crosslinking reaction. Then, the resin was collected and washed to obtain 20 g of an immobilized hydantoinase.

### Reference Example 9

### Conversion of 5-(p-hydroxyphenyl)hydantoin into D-carbamoyl-(p-hydroxyphenyl)glycine by immobilized enzyme

Five grams of the immobilized hydantoinase obtained in Reference Example 8 was added to 100 ml of aqueous 1 mM manganese sulfate at 40°C which was bubbled with nitrogen. To this was added 3 g of 5-(p-hydroxyphenyl)hydantoin and the reaction was carried out with stirring at 40°C for 3 hours with bubbling nitrogen. After completion of the reaction, the reaction mixture was allowed to stand and sucked off to collect the reaction mixture. According to the same manner as described in Example 8, the carbamoyl amino acid product was purified to obtain 2.2 g of D-N-carbamoyl-(p-hydroxyphenyl)glycine.

### Reference Example 10

### Expression of Bacillus sp. KNK245 hydantoinase gene in Bacillus subtilis

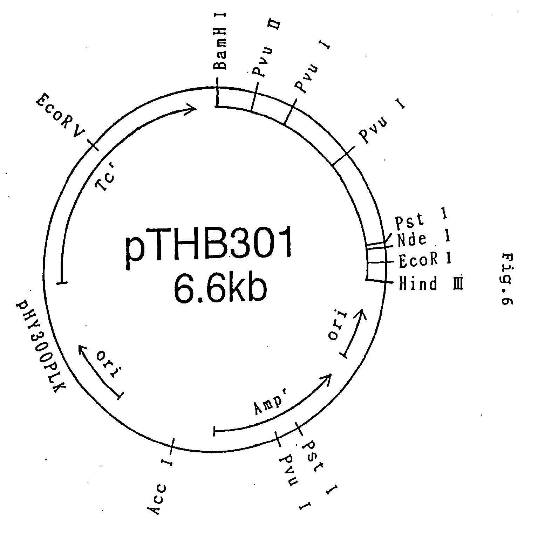

According to the same manner as described in Reference Example 4, PCR was carried out by using the genomic DNA of Bacillus sp. KNK245 as a template and using primers 12 and 13 (Table 1) to obtain a 1.7 kb fragment. This was cleaved with BamHI and HindIII and ligated to the plasmid pHY300PLK (Takara Shuzo) which had been cleaved in a similar manner to obtain the plasmid pTHB301. Its restriction enzyme map is shown in Fig. 6.

This plasmid pTHB301 was introduced into Bacillus subtilis ISW1214 (available from Takara Shuzo), Bacillus subtilis YS-11 (IAM12019) or Bacillus subtilis 3115 (IAM12020) by electroporation (using Shimadzu Corporation GTE-10 Model, 10 KV/cm, 2 ms) and cultivated in the culture medium as shown in Example 4 to which 0.02 g/liter of manganese chloride tetrahydrate was added at 37°C for 20 hours. A cell-free extraction was prepared by collecting the microbial cells and disrupting them with ultrasonication. As a result, each hydantoinase activity was about 2.2 times higher than that obtained by cultivation of Bacillus sp. KNK245 under the same conditions.

### Reference Example 11

### Expression of Bacillus sp. KNK245 hydantoinase gene in thermophile

Bacillus stearothermophilus IFO12550 was transformed with the plasmid pTHB301 prepared in Reference Example 10 by protoplast method according to J. Bacteriol., 149, 824 (1982) and cultivated according to the same manner as described in Example 11 to prepare a cell-free extract. The hydantoinase activity was about 3.6 times higher than that of Bacillus sp. KNK245 prepared under the same conditions.

As described hereinabove, according to the present invention, a microorganism having very high hydantoinase activity can be created and, by using this as a source of enzyme, D-N-carbamoyl-α-amino acids can be produced, efficiently.

### Deposit of Microorganisms

Agrobacterium sp. KNK712 has been deposited as of May 31, 1988 under the accession number of FERM BP-1900; Pseudomonas sp. KNK003A (not part of the invention) has been deposited as of December 1, 1990 under the accession number of FERM BP-3181; Bacillus sp. KNK245 (not part of the invention) has been deposited as of November 2, 1994 under the accession number of FERM BP-4863; and Escherichia coli HB101 pAH1043 has been deposited as of November 2, 1994 under the accession number of FERM BP-4865 at the following Depositary Authority under Budapest Treaty, respectively.
Name: National Institute of Bioscience and Human-Technology (formerly Fermentation Research Institute), Agency of Industrial Science & Technology, Ministry of International Trade & Industry
Address: 1-3, Higashi 1 chome Tsukuba-shi Ibaraki-ken 305, Japan

## Claims

1. A transformant microorganism comprising a recombinant DNA of a DNA fragment containing a gene encoding an enzyme which converts 5-substituted hydantoins into corresponding D-N-carbamoyl-α-amino acids by asymmetric cleavage hydrolysis (hereinafter referred to as hydantoinase) derived from Agrobacterium sp. KNK712 deposited as FERM BP-1900 and a vector DNA.

2. A transformant microorganism according to claim 1, wherein the host microorganism is selected from a microorganism belonging to the genus Escherichia, Pseudomonas, Flavobacterium, Bacillus, Serratia, Agrobacterium, Corynebacterium or Brevibacterium.

3. A transformant microorganism according to claim 2, wherein the transformant microorganism is Escherichia coli HB101 pAH1043 deposited as FERM BP-4865.

4. A process for producing an enzyme which converts 5-substituted hydantoins into corresponding D-N-carbamoyl-α-amino acids by asymmetric cleavage hydrolysis thereof which comprises using the transformant microorganism according to any one of claims 1 to 3.

5. A process for producing D-N-carbamoyl-α-amino acids which comprises using the enzyme obtained by a process according to claim 4.

6. A process according to claim 5, wherein the 5-substituted hydantoin is a compound represented by the general formula (1): 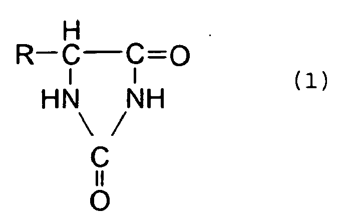 wherein R is phenyl, phenyl substituted with hydroxy, alkyl, substituted alkyl, aralkyl or thienyl.

7. A recombinant plasmid obtained by recombination of a DNA fragment encoding hydantoinase derived from Agrobacterium sp. KNK712 deposited as FERM BP-1900 and plasmid pUC18, and having the restriction enzyme map of Fig. 1.

8. A gene for a protein having an enzymatic activity for conversion of 5-substituted hydantoins into corresponding D-N-carbamoyl-α-amino acids by asymmetric cleavage hydrolysis thereof, said gene encoding the amino acid sequence shown in SEQ ID No. 2.

9. A DNA fragment containing a gene for a protein having an enzymatic activity for conversion of 5-substituted hydantoins into corresponding D-N-carbamoyl-α-amino acids by asymmetric cleavage hydrolysis thereof, said gene having the nucleotide sequence of nucleotide position 160 to position 1533 shown in SEQ ID No. 1 orencoding an amino acid sequence corresponding to the nucleotide sequence of nucleotide position 160 to position 1533 shown in SEQ ID No. 1.

10. An enzyme protein containing the amino acid sequence shown in SEQ ID No. 2 and having an enzymatic activity for conversion of 5-substituted hydantoins into corresponding D-N-carbamoyl-α-amino acids by asymmetric cleavage hydrolysis thereof.

## Patentansprüche

1. Mikroorganismen-Transformante, umfassend eine rekombinante DNA eines DNA-Fragments, das ein Gen enthält, welches für ein Enzym codiert, welches 5-substituierte Hydantoine in die entsprechenden D-N-Carbamoyl-α-aminosäuren durch asymmetrische Hydrolysespaltung umwandelt (nachfolgend bezeichnet als Hydantoinase), welche aus Agrobacterium sp. KNK712 stammt, hinterlegt als FERM BP-1900, und eine Vektor-DNA.

2. Mikroorganismen-Transformante gemäß Anspruch 1, wobei der Wirtsmikroorganismus ausgewählt ist aus einem Mikroorganismus, der zum Genus Escherichia, Pseudomonas, Flavobacterium, Bacillus, Serratia, Agrobacterium, Corynebacterium oder Brevibacterium gehört.

3. Mikroorganismen-Transformante gemäß Anspruch 2, wobei die Mikroorganismen-Transformante Escherichia coli HB101 pAH1043 ist, hinterlegt als FERM BP-4865.

4. Verfahren zum Herstellen eines Enzyms, das 5-substituierte Hydantoine in die entsprechenden D-N-Carbamoyl-α-aminosäuren durch asymmetrische Hydrolysespaltung davon umwandelt, welches die Verwendung der Mikroorganismen-Transformante gemäß irgendeinem der Ansprüche 1 bis 3 umfaßt.

5. Verfahren zum Herstellen von D-N-Carbamoyl-α-aminosäuren, welches die Verwendung des Enzyms umfaßt, welches in einem Verfahren gemäß Anspruch 4 gewonnen wurde.

6. Verfahren gemäß Anspruch 5, wobei das 5-substituierte Hydantoin eine Verbindung ist, die durch die allgemeine Formel (1) dargestellt wird: 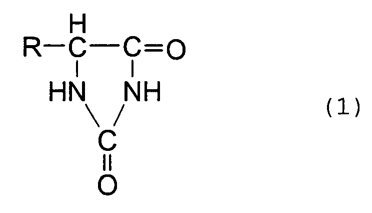 worin R Phenyl ist, Phenyl, welches mit Hydroxy substituiert ist, Alkyl, substituiertes Alkyl, Aralkyl oder Thienyl.

7. Rekombinantes Plasmid, das durch eine Rekombination eines DNA-Fragments, welches für eine Hydantoinase codiert, die aus Agrobacterium sp. KNK712, hinterlegt als FERM BP-1900, und dem Plasmid pUC18 gewonnen wird, und die Restriktionsenzymkarte in Fig. 1 hat.

8. Gen für ein Protein, welches die enzymatische Aktivität zur Umwandlung von 5-substituierten Hydantoinen in die entsprechenden D-N-Carbamoyl-α-aminosäuren durch asymmetrische Hydrolysespaltung davon besitzt, wobei das Gen eine Aminosäuresequenz codiert, die in SEQ ID No. 2 gezeigt ist.

9. DNA-Fragment, welches ein Gen für ein Protein enthält, das die enzymatische Aktivität zur Umwandlung von 5-substituierten Hydantoinen in entsprechende D-N-Carbamoyl-α-aminosäuren durch asymmetrische Hydrolysespaltung davon hat, wobei das Gen die Nukleotidsequenz von Nukleotidposition 160 bis Position 1533 hat, welche in SEQ ID No. 1 gezeigt ist, oder eine Aminosäuresequenz codiert, die der Nukleotidsequenz von Nukleotidposition 160 bis Position 1533 entspricht, die in SEQ ID No. 1 gezeigt ist.

10. Enzymprotein, welches die Aminosäuresequenz enthält, die in SEQ ID No. 2 gezeigt ist, und die enzymatische Aktivität zur Umwandlung von 5-substituierten Hydantoinen in entsprechende D-N-Carbamoyl-α-aminosäuren durch asymmetrische Hydrolysespaltung davon hat.

## Revendications

1. Microorganisme transformant comprenant un ADN recombinant d'un fragment d'ADN contenant un gène codant pour une enzyme qui convertit des hydantoïnes substituées en position 5 en acides D-N-carbamoyl-α-aminés correspondants par hydrolyse par clivage asymétrique (à laquelle on se réfère ci-dessous par hydantoïnase), dérivée de d'Agrobacterium sp. KNK712 déposé en tant que FERM BP-1900 et un vecteur d'ADN.

2. Microorganisme transformant selon la revendication 1, le microorganisme hôte étant sélectionné parmi des microorganismes appartenant aux genres Escherichia, Pseudomonas, Flavobacterium, Bacillus, Serratia, Agrobacterium, Corynebacterium ou Brevibacterium.

3. Microorganisme transformant selon la revendication 2, lequel microorganisme transformant est Escherichia coli HB101 pAH1043 déposé en tant que FERM BP-4865.

4. Procédé pour produire une enzyme qui convertit des hydantoïnes substituées en position 5 en acides D-N-carbamoyl-α-aminés correspondants par hydrolyse par clivage asymétrique de celles-ci, qui comprend l'utilisation du microorganisme transformant selon l'une quelconque des revendications 1 à 3.

5. Procédé pour produire des acides D-N-carbamoyl-α-aminés, qui comprend l'utilisation de l'enzyme obtenue par un procédé selon la revendication 4.

6. Procédé selon la revendication 5, dans lequel l'hydantoïne substituée en position 5 est un composé représenté par la formule générale (1) : 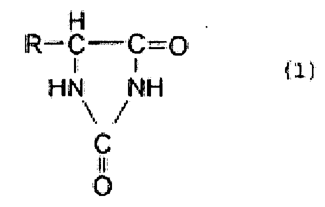 dans laquelle R représente un groupe phényle, phényle substitué par un groupe hydroxy, alkyle, alkyle substitué, aralkyle ou thiényle.

7. Plasmide recombinant obtenu par recombinaison d'un fragment d'ADN codant une hydantoïnase dérivée d'Agrobacterium sp. KNK712 déposé en tant que FERM BP-1900 et du plasmide pUC 18 ayant la carte de restriction enzymatique de la figure 1.

8. Gène pour une protéine ayant une activité enzymatique pour la conversion d'hydantoïnes substituées en position 5 en acides D-N-carbamoyl-α-aminés correspondants par hydrolyse par clivage asymétrique de celles-ci, ledit gène codant pour la séquence d'acides aminés présentée dans SEQ ID No. 2.

9. Fragment d'ADN contenant un gène pour une protéine ayant une activité enzymatique pour la conversion d'hydantoïnes substituées en position 5 en acides D-N-carbamoyl-α-aminés correspondants par hydrolyse pour clivage asymétrique de celles-ci, ledit gène ayant la séquence de nucléotides de la position du nucléotide 160 à la position 1533 présentée dans SEQ ID No. 1 ou codant pour la séquence d'acides aminés correspondant à la séquence de nucléotides de la position du nucléotide 160 à la position 1533 présentée dans SEQ ID No. 1.

10. Protéine enzymatique contenant la séquence d'acides aminés présentée dans SEQ ID No. 2 et ayant une activité enzymatique de conversion d'hydantoïnes substituées en position 5 en acides D-N-carbamoyl-a-aminés correspondants par hydrolyse par clivage asymétrique de celles-ci.


### SEQUENCE LISTING

<110> KANEGAFUCHI KAGAKU KOGYO KABUSHIKI KAISHA
<120> PROCESS FOR PRODUCING D-N-CARBAMOYL-ALPHA-AMINO ACID
<130> 660298
<150> JP 1994-326865
   <151> 1994-12-28
<160> 4
<170> PatentIn version 3.2
<210> 1
   <211> 2105
   <212> DNA
   <213> Agrobacterium sp.
<220>
   <221> CDS
   <222> (160)..(1533)
<400> 1
   gtcgacttca actcgttccg cctcgaacga gagcgctctg cacagaatcg tcttcgctag      60
   gccgccgttc cgcttgacag cttcaaaaca ccatgcaaac ttgtataaag aattacattc     120
   catatacgga acaaactctt caatcctgag agcgacatc atg gac atc att atc       174
                                              Met Asp Ile Ile Ile
                                              1               5
   aaa aac gga acc atc gtg acc gcg gat ggc att tct cgc gcc gat ctc      222
   Lys Asn Gly Thr Ile Val Thr Ala Asp Gly Ile Ser Arg Ala Asp Leu
                   10                  15                  20
   ggg atc aag gat ggc aag atc acc cag atc ggc ggc gcg ctc ggc cca      270
   Gly Ile Lys Asp Gly Lys Ile Thr Gln Ile Gly Gly Ala Leu Gly Pro
               25                  30                  35
   gcg gag cgg acg atc gac gcg gcc ggc cgc tac gtc ttt ccg ggc ggc      318
   Ala Glu Arg Thr Ile Asp Ala Ala Gly Arg Tyr Val Phe Pro Gly Gly
           40                  45                  50
   ata gac gtt cac acg cat gtc gaa acg gtc agc ttc aac acg cag tcg      366
   Ile Asp Val His Thr His Val Glu Thr Val Ser Phe Asn Thr Gln Ser
       55                  60                  65
   gcc gac acg ttc gca aca gcg acg gtc gcg gcc gcc tgt ggc gga acg      414
   Ala Asp Thr Phe Ala Thr Ala Thr Val Ala Ala Ala Cys Gly Gly Thr
   70                  75                  80                  85
   aca acc atc gtc gat ttc tgt cag cag gat cgc ggc cac agc ctg gcg      462
   Thr Thr Ile Val Asp Phe Cys Gln Gln Asp Arg Gly His Ser Leu Ala
                   90                  95                  100
   gaa ccc gtc ccc aag tgg gac ggt atg gcc ggc ggc aag tcg gcg atc      510
   Glu Pro Val Pro Lys Trp Asp Gly Met Ala Gly Gly Lys Ser Ala Ile
               105                 110                 115
   gat tac ggc tac cac atc atc gtg ctc gac ccg acc gac agc gtg att      558
   Asp Tyr Gly Tyr His Ile Ile Val Leu Asp Pro Thr Asp Ser Val Ile
           120                 125                 130
   gag gag ctg gag gtg ctt ccc gat ctt ggc att acc tcc ttc aag gtc      606
   Glu Glu Leu Glu Val Leu Pro Asp Leu Gly Ile Thr Ser Phe Lys Val
       135                 140                 145
   ttc atg gcc tat cgc ggc atg aac atg atc gac gac ctg acg ctg ctg      654
   Phe Met Ala Tyr Arg Gly Met Asn Met Ile Asp Asp Leu Thr Leu Leu
   150                 155                 160                 165
   aag acg ctc gac aag gcg gtc aag acc gga tcg ctc gtc atg gtg cac      702
   Lys Thr Leu Asp Lys Ala Val Lys Thr Gly Ser Leu Val Met Val His
                   170                 175                 180
   gcg gaa aac ggc gac gcc gcc gac tat ctg cgc gac aag ttc gtg gcc 750
   Ala Glu Asn Gly Asp Ala Ala Asp Tyr Leu Arg Asp Lys Phe Val Ala
   185 190 195
   gag ggc aaa acc gcg ccg atc tac cac gcg ctc agc cgc ccg ccc cgg 798
   Glu Gly Lys Thr Ala Pro Ile Tyr His Ala Leu Ser Arg Pro Pro Arg
   200 205 210
   gtc gaa gcc gag gca acc gcg cgg gcc ctc gcc ctg gcc gaa atc gtc 846
   Val Glu Ala Glu Ala Thr Ala Arg Ala Leu Ala Leu Ala Glu Ile Val
   215 220 225
   aac gcc ccg atc tac ata gtc cat gtg acc tgc gag gag tcc ctt gag 894
   Asn Ala Pro Ile Tyr Ile Val His Val Thr Cys Glu Glu Ser Leu Glu
   230 235 240 245
   gag gtg atg cgc gca aaa tcg cga ggc gtc cgc gct ctg gcg gaa acc 942
   Glu Val Met Arg Ala Lys Ser Arg Gly Val Arg Ala Leu Ala Glu Thr
   250 255 260
   tgc acg cat tac ctt tac ctc acc aag gaa gac ctg gag cgg ccg gat 990
   Cys Thr His Tyr Leu Tyr Leu Thr Lys Glu Asp Leu Glu Arg Pro Asp
   265 270 275
   ttc gaa ggt gcg aaa tac gtt ttc aca ccg ccg gcc cgc gcg aag aaa 1038
   Phe Glu Gly Ala Lys Tyr Val Phe Thr Pro Pro Ala Arg Ala Lys Lys
   280 285 290
   gac cat gac gtt ctc tgg aac gca ctc aga aac ggt gtg ttc gaa acg 1086
   Asp His Asp Val Leu Trp Asn Ala Leu Arg Asn Gly Val Phe Glu Thr
   295 300 305
   gtt tcc tcc gac cat tgc tcc tgg ctc ttc aag ggg cac aag gac cgg 1134
   Val Ser Ser Asp His Cys Ser Trp Leu Phe Lys Gly His Lys Asp Arg
   310 315 320 325
   ggc cgg aac gac ttt cgc gcc atc ccg aac ggc gcg ccg ggc gtc gag 1182
   Gly Arg Asn Asp Phe Arg Ala Ile Pro Asn Gly Ala Pro Gly Val Glu
   330 335 340
   gaa cgg ttg atg atg gtc tat cag ggc gtc aac gaa ggc cgg att tcc      1230
   Glu Arg Leu Met Met Val Tyr Gln Gly Val Asn Glu Gly Arg Ile Ser
                   345                     350                     355
   ctt acc cag ttc gtg gaa ctg gtc gcc acg cgc ccg gcc aag gtc ttc      1278
   Leu Thr Gln Phe Val Glu Leu Val Ala Thr Arg Pro Ala Lys Val Phe
               360                     365                     370
   gga atg ttt ccg caa aag ggg acg atc gcg gtc ggt tcg gac gcc gac      1326
   Gly Met Phe Pro Gln Lys Gly Thr Ile Ala Val Gly Ser Asp Ala Asp
           375                     380                     385
   atc gtc ctt tgg gac ccc gag gcc gaa atg gtg atc gaa cag acc gcc      1374
   Ile Val Leu Trp Asp Pro Glu Ala Glu Met Val Ile Glu Gln Thr Ala
   390                     395                     400                     405
   atg cac aac gcc atg gat tac tcc tcc tac gag gga cac aag gtc aag      1422
   Met His Asn Ala Met Asp Tyr Ser Ser Tyr Glu Gly His Lys Val Lys
                   410                     415                     420
   ggc gtg ccg aag acg gtg ctc ctg cgt ggc aag gtt atc gtc gac gaa      1470
   Gly Val Pro Lys Thr Val Leu Leu Arg Gly Lys Val Ile Val Asp Glu
               425                     430                     435
   ggt tcc tat gtc ggc gaa ccg acg gac ggg aaa ttc ctg aaa cgt cgc      1518
   Gly Ser Tyr Val Gly Glu Pro Thr Asp Gly Lys Phe Leu Lys Arg Arg
           440                     445                     450
   aaa tac aag cag taa accggcattc tacgagaacg atgatgacaa tacacacttc      1573
   Lys Tyr Lys Gln
       455
   gacaagtgtc gtcgcgcctg acggcgacag cgttgcaagc ggcttcgaca ggcgttacgg    1633
   cgcaccgaca ttcaatcaga taccatcggg cgaacggctg gtgtcggact gcatcgcgct    1693
   gatgctgaag caccgttccg tcagacgcta cacgggggaa cctctccccc caggcacgct    1753
   ggaaatgctg atggcggcgg gtcagtcggc tgcgacatcc tccaacatgc agaccgtgtc   1813
   ggtcatcgcg gtgacggacc ccgagaaaaa gacccgtctc gcgaagacat gcgccgggca   1873
   ggacttcatc gccaacgctc ctgtgctcct gtgtttcgtg acggatcttg cgcgccccgc   1933
   cagggtcgcc tccacgattg gcgccgatct tttcgcgttg ccgatgatcg acacctttct   1993
   ggcgtccatc agcgattgtt ccatcttcgc gcaaaacctt gttctcgccg cggaatcgct   2053
   cggcatgggc acctgctatg tcgggagcct gcgcaaccgg cctgatcccg gg           2105
<210> 2
   <211> 457
   <212> PRT
   <213> Agrobacterium sp.
<400> 2
   Met Asp Ile Ile Ile Lys Asn Gly Thr Ile Val Thr Ala Asp Gly Ile
   1               5                   10                  15
   Ser Arg Ala Asp Leu Gly Ile Lys Asp Gly Lys Ile Thr Gln Ile Gly
               20                  25                  30
   Gly Ala Leu Gly Pro Ala Glu Arg Thr Ile Asp Ala Ala Gly Arg Tyr
           35                  40                  45
   Val Phe Pro Gly Gly Ile Asp Val His Thr His Val Glu Thr Val Ser
       50                  55                  60
   Phe Asn Thr Gln Ser Ala Asp Thr Phe Ala Thr Ala Thr Val Ala Ala
   65                  70                  75                  80
   Ala Cys Gly Gly Thr Thr Thr Ile Val Asp Phe Cys Gln Gln Asp Arg
   85 90 95
   Gly His Ser Leu Ala Glu Pro Val Pro Lys Trp Asp Gly Met Ala Gly
   100 105 110
   Gly Lys Ser Ala Ile Asp Tyr Gly Tyr His Ile Ile Val Leu Asp Pro
   115 120 125
   Thr Asp Ser Val Ile Glu Glu Leu Glu Val Leu Pro Asp Leu Gly Ile
   130 135 140
   Thr Ser Phe Lys Val Phe Met Ala Tyr Arg Gly Met Asn Met Ile Asp
   145 150 155 160
   Asp Leu Thr Leu Leu Lys Thr Leu Asp Lys Ala Val Lys Thr Gly Ser
   165 170 175
   Leu Val Met Val His Ala Glu Asn Gly Asp Ala Ala Asp Tyr Leu Arg
   180 185 190
   Asp Lys Phe Val Ala Glu Gly Lys Thr Ala Pro Ile Tyr His Ala Leu
   195 200 205
   Ser Arg Pro Pro Arg Val Glu Ala Glu Ala Thr Ala Arg Ala Leu Ala
   210 215 220
   Leu Ala Glu Ile Val Asn Ala Pro Ile Tyr Ile Val His Val Thr Cys
   225 230 235 240
   Glu Glu Ser Leu Glu Glu Val Met Arg Ala Lys Ser Arg Gly Val Arg
   245 250 255
   Ala Leu Ala Glu Thr Cys Thr His Tyr Leu Tyr Leu Thr Lys Glu Asp
   260 265 270
   Leu Glu Arg Pro Asp Phe Glu Gly Ala Lys Tyr Val Phe Thr Pro Pro
   275 280 285
   Ala Arg Ala Lys Lys Asp His Asp Val Leu Trp Asn Ala Leu Arg Asn
   290 295 300
   Gly Val Phe Glu Thr Val Ser Ser Asp His Cys Ser Trp Leu Phe Lys
   305 310 315 320
   Gly His Lys Asp Arg Gly Arg Asn Asp Phe Arg Ala Ile Pro Asn Gly
   325 330 335
   Ala Pro Gly Val Glu Glu Arg Leu Met Met Val Tyr Gln Gly Val Asn
   340 345 350
   Glu Gly Arg Ile Ser Leu Thr Gln Phe Val Glu Leu Val Ala Thr Arg
   355 360 365
   Pro Ala Lys Val Phe Gly Met Phe Pro Gln Lys Gly Thr Ile Ala Val
   370 375 380
   Gly Ser Asp Ala Asp Ile Val Leu Trp Asp Pro Glu Ala Glu Met Val
   385                 390                 395                 400
   Ile Glu Gln Thr Ala Met His Asn Ala Met Asp Tyr Ser Ser Tyr Glu
                   405                 410                 415
   Gly His Lys Val Lys Gly Val Pro Lys Thr Val Leu Leu Arg Gly Lys
               420                 425                 430
   Val Ile Val Asp Glu Gly Ser Tyr Val Gly Glu Pro Thr Asp Gly Lys
           435                 440                 445
   Phe Leu Lys Arg Arg Lys Tyr Lys Gln
       450                 455
<210> 3
   <211> 1569
   <212> DNA
   <213> Pseudomonas sp.
<220>
   <221> CDS
   <222> (60)..(1517)
<400> 3
   tcggaacctg aaaacgaaat atcccgccgg acggtgggaa ggtgaaagga ggagtctcc      59
   atg aca aca gtt atc aag ggt gga acg atc gtc gcc gcc gat cgc agc      107
   Met Thr Thr Val Ile Lys Gly Gly Thr Ile Val Ala Ala Asp Arg Ser
   1               5                   10                  15
   tat gaa gcc gat atc ctg atc gaa ggc gaa aag atc gcc cag atc ggc      155
   Tyr Glu Ala Asp Ile Leu Ile Glu Gly Glu Lys Ile Ala Gln Ile Gly
               20                  25                  30
   agg gat ctg cag ggc gac aag att gta gac gcc gag ggt gcc tat atc      203
   Arg Asp Leu Gln Gly Asp Lys Ile Val Asp Ala Glu Gly Ala Tyr Ile
           35                  40                  45
   att ccc ggc ggc atc gat cct cac acg cat ctt gaa atg ccc ttt atg      251
   Ile Pro Gly Gly Ile Asp Pro His Thr His Leu Glu Met Pro Phe Met
       50                  55                  60
   ggc acg acc act gcc gag acc tgg gag tcc ggc act ttc gcg gct ctc      299
   Gly Thr Thr Thr Ala Glu Thr Trp Glu Ser Gly Thr Phe Ala Ala Leu
   65                  70                  75                  80
   tcc ggc ggc acc acg atg gtt gtg gat ttc gtc att ccc ggg ccg gcg      347
   Ser Gly Gly Thr Thr Met Val Val Asp Phe Val Ile Pro Gly Pro Ala
                   85                  90                  95
   gga atg ctt gcc gcc ttc gat caa tgg cag gag agg gca gcg cgc cag      395
   Gly Met Leu Ala Ala Phe Asp Gln Trp Gln Glu Arg Ala Ala Arg Gln
               100                 105                 110
   gct tcg tcc gac tac tcg ctg cat atg tgc gtg acc ggc tgg tca aag      443
   Ala Ser Ser Asp Tyr Ser Leu His Met Cys Val Thr Gly Trp Ser Lys
           115                 120                 125
   cag atc ttc gag gac atg gcg aag gtg gtc gag cgc ggt gtc aac acc      491
   Gln Ile Phe Glu Asp Met Ala Lys Val Val Glu Arg Gly Val Asn Thr
       130                 135                 140
   ttc aag cat ttc atg gcc tat aag ggc gcg ctg atg gtg aac gac gac      539
   Phe Lys His Phe Met Ala Tyr Lys Gly Ala Leu Met Val Asn Asp Asp
   145                 150                 155                 160
   gag atg ttc gcc tcc ttc cag cgc tgc gcc gcc ctg ggc gcc ctc ccg      587
   Glu Met Phe Ala Ser Phe Gln Arg Cys Ala Ala Leu Gly Ala Leu Pro
                   165                 170                 175
   ctc gtg cat gcg gaa aac ggc gac atc gtc gcc tcg ctg cag cag aaa      635
   Leu Val His Ala Glu Asn Gly Asp Ile Val Ala Ser Leu Gln Gln Lys
   180 185 190
   tat atg cag gaa ggc ctc acg ggt ccg gag gct cat gcc tat tcc cgc 683
   Tyr Met Gln Glu Gly Leu Thr Gly Pro Glu Ala His Ala Tyr Ser Arg
   195 200 205
   ccg ccg gag gtc gag ggc gag gcc acc aac cgc gcc atc atg atc gcc 731
   Pro Pro Glu Val Glu Gly Glu Ala Thr Asn Arg Ala Ile Met Ile Ala
   210 215 220
   gat gcc gca ggc gtg ccg gtc tat atc gtg cat gtc tcc tgt gaa cag 779
   Asp Ala Ala Gly Val Pro Val Tyr Ile Val His Val Ser Cys Glu Gln
   225 230 235 240
   gcg cac gaa gcg atc cgc cgc gca cgc cag aag gga atg cgc gtc tat 827
   Ala His Glu Ala Ile Arg Arg Ala Arg Gln Lys Gly Met Arg Val Tyr
   245 250 255
   ggc gag ccg ctc atc cag cac ctg ctg ctt gac gag tcc gag tac aag 875
   Gly Glu Pro Leu Ile Gln His Leu Leu Leu Asp Glu Ser Glu Tyr Lys
   260 265 270
   aga gcc gac tgg gac gag gcg gct cgg cgg gtg atg tcc gcg cca ttc 923
   Arg Ala Asp Trp Asp Glu Ala Ala Arg Arg Val Met Ser Ala Pro Phe
   275 280 285
   cgc gat aaa agc cat cag gac agc ctc tgg gct ggg ctc gca gcg ggc 971
   Arg Asp Lys Ser His Gln Asp Ser Leu Trp Ala Gly Leu Ala Ala Gly
   290 295 300
   tcg ctg cag gtc gtg gca acg gac cac tgc gcc ttt acc act gaa cag 1019
   Ser Leu Gln Val Val Ala Thr Asp His Cys Ala Phe Thr Thr Glu Gln
   305 310 315 320
   aag cgg ctg gga ctc aac gac ttc acg aaa atc ccg aac ggc acc ggg 1067
   Lys Arg Leu Gly Leu Asn Asp Phe Thr Lys Ile Pro Asn Gly Thr Gly
   325 330 335
   ggg tta gag gac cgg ttg tcg ctg ctc tgg acc tat ggc gtg aag acg 1115
   Gly Leu Glu Asp Arg Leu Ser Leu Leu Trp Thr Tyr Gly Val Lys Thr
               340                 345                 350
   ggg cgg ctc acg cct aac gaa ttc gtc gcc gtc acc tcc acc aat atc      1163
   Gly Arg Leu Thr Pro Asn Glu Phe Val Ala Val Thr Ser Thr Asn Ile
           355                 360                 365
   gca caa atc ctg aac atc tat ccg caa aag ggc gca atc ctg cct ggg      1211
   Ala Gln Ile Leu Asn Ile Tyr Pro Gln Lys Gly Ala Ile Leu Pro Gly
       370                 375                 380
   tcc gat gcc gac ctc gtc gtc tgg gat cca gcc aaa tcg aag aag atc      1259
   Ser Asp Ala Asp Leu Val Val Trp Asp Pro Ala Lys Ser Lys Lys Ile
   385                 390                 395                 400
   act gct tcc gct cag aaa tcc atc atc gac tac aat atc ttc gag ggc      1307
   Thr Ala Ser Ala Gln Lys Ser Ile Ile Asp Tyr Asn Ile Phe Glu Gly
                   405                 410                 415
   tac gag gtg acc ggc atg ccg cga tac acg ctc tca cgc ggc gag gtc      1355
   Tyr Glu Val Thr Gly Met Pro Arg Tyr Thr Leu Ser Arg Gly Glu Val
               420                 425                 430
   gta tgg ggc gag gaa ggc agc aac gaa ccg cgg ccc ggt cgc ggt agg      1403
   Val Trp Gly Glu Glu Gly Ser Asn Glu Pro Arg Pro Gly Arg Gly Arg
           435                 440                 445
   ttc gtc gag cgc cgg ccc ttt gcc gcc gtc agc cgt gcc ctt tca acc      1451
   Phe Val Glu Arg Arg Pro Phe Ala Ala Val Ser Arg Ala Leu Ser Thr
       450                 455                 460
   tgg aag gag atc gtc gct ccg cgt gcg gtg gag cgc tcg gca aag cat      1499
   Trp Lys Glu Ile Val Ala Pro Arg Ala Val Glu Arg Ser Ala Lys His
   465                 470                 475                 480
   atg ccg atc ggc gtt tga attttgggag gcaggctcga actacggttt             1547
   Met Pro Ile Gly Val
                   485
   ggcctgtctc aggacaatcc at 1569
<210> 4
   <211> 485
   <212> PRT
   <213> Pseudomonas sp.
<400> 4
   Met Thr Thr Val Ile Lys Gly Gly Thr Ile Val Ala Ala Asp Arg Ser
   1 5 10 15
   Tyr Glu Ala Asp Ile Leu Ile Glu Gly Glu Lys Ile Ala Gln Ile Gly
   20 25 30
   Arg Asp Leu Gln Gly Asp Lys Ile Val Asp Ala Glu Gly Ala Tyr Ile
   35 40 45
   Ile Pro Gly Gly Ile Asp Pro His Thr His Leu Glu Met Pro Phe Met
   50 55 60
   Gly Thr Thr Thr Ala Glu Thr Trp Glu Ser Gly Thr Phe Ala Ala Leu
   65 70 75 80
   Ser Gly Gly Thr Thr Met Val Val Asp Phe Val Ile Pro Gly Pro Ala
   85 90 95
   Gly Met Leu Ala Ala Phe Asp Gln Trp Gln Glu Arg Ala Ala Arg Gln
   100 105 110
   Ala Ser Ser Asp Tyr Ser Leu His Met Cys Val Thr Gly Trp Ser Lys
   115 120 125
   Gln Ile Phe Glu Asp Met Ala Lys Val Val Glu Arg Gly Val Asn Thr
   130 135 140
   Phe Lys His Phe Met Ala Tyr Lys Gly Ala Leu Met Val Asn Asp Asp
   145 150 155 160
   Glu Met Phe Ala Ser Phe Gln Arg Cys Ala Ala Leu Gly Ala Leu Pro
   165 170 175
   Leu Val His Ala Glu Asn Gly Asp Ile Val Ala Ser Leu Gln Gln Lys
   180 185 190
   Tyr Met Gln Glu Gly Leu Thr Gly Pro Glu Ala His Ala Tyr Ser Arg
   195 200 205
   Pro Pro Glu Val Glu Gly Glu Ala Thr Asn Arg Ala Ile Met Ile Ala
   210 215 220
   Asp Ala Ala Gly Val Pro Val Tyr Ile Val His Val Ser Cys Glu Gln
   225 230 235 240
   Ala His Glu Ala Ile Arg Arg Ala Arg Gln Lys Gly Met Arg Val Tyr
   245 250 255
   Gly Glu Pro Leu Ile Gln His Leu Leu Leu Asp Glu Ser Glu Tyr Lys
   260 265 270
   Arg Ala Asp Trp Asp Glu Ala Ala Arg Arg Val Met Ser Ala Pro Phe
   275 280 285
   Arg Asp Lys Ser His Gln Asp Ser Leu Trp Ala Gly Leu Ala Ala Gly
   290 295 300
   Ser Leu Gln Val Val Ala Thr Asp His Cys Ala Phe Thr Thr Glu Gln
   305 310 315 320
   Lys Arg Leu Gly Leu Asn Asp Phe Thr Lys Ile Pro Asn Gly Thr Gly
   325 330 335
   Gly Leu Glu Asp Arg Leu Ser Leu Leu Trp Thr Tyr Gly Val Lys Thr
   340 345 350
   Gly Arg Leu Thr Pro Asn Glu Phe Val Ala Val Thr Ser Thr Asn Ile
   355 360 365
   Ala Gln Ile Leu Asn Ile Tyr Pro Gln Lys Gly Ala Ile Leu Pro Gly
   370 375 380
   Ser Asp Ala Asp Leu Val Val Trp Asp Pro Ala Lys Ser Lys Lys Ile
   385 390 395 400
   Thr Ala Ser Ala Gln Lys Ser Ile Ile Asp Tyr Asn Ile Phe Glu Gly
   405 410 415
   Tyr Glu Val Thr Gly Met Pro Arg Tyr Thr Leu Ser Arg Gly Glu Val
   420 425 430
   Val Trp Gly Glu Glu Gly Ser Asn Glu Pro Arg Pro Gly Arg Gly Arg
   435 440 445
   Phe Val Glu Arg Arg Pro Phe Ala Ala Val Ser Arg Ala Leu Ser Thr
   450 455 460
   Trp Lys Glu Ile Val Ala Pro Arg Ala Val Glu Arg Ser Ala Lys His
   465 470 475 480
   Met Pro Ile Gly Val
   485